(19) Europäisches Patentamt
European Patent Office
Office européen des brevets

(11) **EP 1 615 680 B1**

(12) **EUROPÄISCHE PATENTSCHRIFT**

(45) Veröffentlichungstag und Bekanntmachung des Hinweises auf die Patenterteilung:
**05.05.2010 Patentblatt 2010/18**

(21) Anmeldenummer: **04711604.1**

(22) Anmeldetag: **17.02.2004**

(51) Int Cl.:
**A61M 1/16** (2006.01)

(86) Internationale Anmeldenummer:
**PCT/EP2004/001457**

(87) Internationale Veröffentlichungsnummer:
**WO 2004/089440 (21.10.2004 Gazette 2004/43)**

(54) **BLUTBEHANDLUNGSVORRICHTUNG**

HAEMODIALYSIS DEVICE

DISPOSITIF D'HEMODIALYSE

(84) Benannte Vertragsstaaten:
**AT BE BG CH CY CZ DE DK EE ES FI FR GB GR HU IE IT LI LU MC NL PT RO SE SI SK TR**

(30) Priorität: **11.04.2003 DE 10317024**

(43) Veröffentlichungstag der Anmeldung:
**18.01.2006 Patentblatt 2006/03**

(73) Patentinhaber: **Fresenius Medical Care Deutschland GmbH**
**61352 Bad Homburg v.d.H. (DE)**

(72) Erfinder:
• **GROSS, Malte**
**97464 Niederwerrn (DE)**

• **WÜPPER, Andreas**
**64572 BÜTTELBORN (DE)**

(74) Vertreter: **Ziermann, Oliver**
**Fresenius Medical Care**
**AG & Co. KGaA**
**Frankfurter Straße 6-8**
**66606 St. Wendel (DE)**

(56) Entgegenhaltungen:
EP-A- 0 911 043      US-A- 5 110 477
US-A- 5 567 320      US-A- 5 744 031
US-B1- 6 325 774

**Beschreibung**

Die Erfindung betrifft das Gebiet von Blutbehandlungsvorrichtungen mit einem Blutreinigungselement nach dem Oberbegriff des Anspruch 1.

In der Nierenersatzbehandlung werden verschiedene Verfahren eingesetzt. Bei einigen dieser Verfahren wird einem Patienten während der Behandlung kontinuierlich Blut entnommen und in einen extrakorporalen Kreislauf eingespeist. Dort durchfließt es ein Blutreinigungselement, um danach zum Patient zurückgeführt zu werden. Das Blutreinigungselement weist meist einen durch eine semipermeable Membran in zwei Kammern geteiltes Filterelement auf, dessen eine Kammer vom Blut durchströmt wird. Gegenwärtig werden hierfür vor allem Filterelemente verwendet, die viele Tausend von Hohlfasern beinhalten, deren Inneres vom Blut durchflossen wird.

Bei der Hämodialyse wird die andere Kammer von einer Reinigungsflüssigkeit (Dialysierflüssigkeit) durchflossen, die aus dem Blut zu entfernende Stoffe wie z.B. Harnstoff per Diffusion aufnimmt und bezüglich anderer, im Blut zu belassender Stoffe wie Elektrolyte eine Zusammensetzung ähnlich eines gesunden Blutbildes aufweist. Auszuscheidene Flüssigkeitsvolumina werden mittels einer Komponente, die die Ultrafiltration steuert, ebenfalls von der Blutkammer zur Dialysierflüssigkeitskammer des Filterelementes entfernt.

Bei der Hämofiltration wird die andere Kammer des Filterelementes, die fortan als erste Kammer bezeichnet wird, nicht durch eine zweite Flüssigkeit vollständig durchflossen. Vielmehr wird in diese Kammer nur Ultrafiltrat über die Membran hinzugeführt, das dann über eine Ultrafiltratableitung abgeführt wird. Dabei wird die entfernte Flüssigkeitsmenge weit über der gehalten, die dem Patienten zur Erreichung seines Trockengewichtes entfernt werden muß. Auf diese Weise werden zu entfernende Stoffe wie Harnstoff in nennenswertem Umfang durch Konvektion mit dem Ultrafiltrat abgeführt. Gleichzeitig wird fast die gesamte Flüssigkeitsmenge durch eine Substitutionsflüssigkeit ersetzt, die dem Patienten an geeigneter Stelle über den extrakorporalen Kreislauf zurückgegeben wird.

Da Konvektion und Diffusion verschiedengroße Moleküle unterschiedlich effektiv durch die Membran entfernen können, wird auch die Kombination beider Verfahren in Form einer Hämodiafiltrationsbehandlung angewendet. Moderne Dialysemaschinen bieten hierzu die Möglichkeit, zwischen diesen Behandlungsmodi zu wechseln, ohne dass es eines komplexen Umbaus bedarf. Dabei weisen einige bekannte Geräte die Möglichkeit auf, die Dialysier- und die Substitutionsflüssigkeit online während der Behandlung aus Wasser und entsprechenden Konzentrat durch die Maschine bereitzustellen. Bei diesen Vorrichtungen ist es nicht mehr notwendig, enorme Mengen dieser Flüssigkeiten (bis ca. 200 Liter) in Form von Beuteln bereitzuhalten. Eine solche Vorrichtung ist z.B. Gegenstand der EP 0 930 080 A1.

Um den Erfolg einer Nierenersatzbehandlung überwachen zu können, ist die Bestimmung von Behandlungsparametern an solchen Blutreinigungsgeräten, insbesondere der Blutreinigungsleistung des Blutreinigungselementes, von großem Interesse. Als Blutreinigungsleistung wird zumeist die Clearance oder Dialysance des Blutreinigungselementes angegeben.

Die Clearance K ist als der Blutstrom definiert, der durch das Blutreinigungselement vollständig von einer Substanz (z.B. Harnstoff) befreit wird. Dabei wird bei einer Hämodialysebehandlung vorausgesetzt, dass die Dialysierflüssigkeit beim Eintritt in den Dialysator die zu entfernende Substanz nicht enthält. Die Clearance ist von Fläche und Material des Dialysators und den jeweiligen Betriebsbedingungen (Blut-, Dialysierflüssigkeits- und Ultrafiltrationsfluss) abhängig. Die Clearance kommt sowohl durch Diffusion als auch durch Konvektion über die Membran des Filterelementes - des Dialysators - zustande.

Der Begriff der Clearance läßt sich auch auf Substanzen wie z.B. Natriumionen erweitern, die bereits in der Dialysierflüssigkeit vorhanden sind. In diesem Fall spricht man von der Dialysance D. Sie ist als der Blutfluss definiert, der vollständig auf das Konzentrationsniveau in der Dialysierflüssigkeit gebracht wird.

Aus der Clearance K kann die dimensionslose Größe Kt/V berechnet werden, wobei t die Behandlungsdauer und V das Verteilungsvolumen der Substanz im menschlichen Körper ist. Kt/V für Harnstoff wird weitverbreitet als Maß für die Effizienz einer Dialysebehandlung angewendet.

Die Messung der Harnstoffkonzentration ist jedoch bisher relativ aufwendig. Entweder erfordert sie die Entnahme von Blutproben, was für den Patienten mit Unannehmlichkeiten einhergeht und zudem keine schnelle, automatisierte Auswertung ermöglicht, oder sie gestaltet sich als Messung in der verbrauchten Dialysierflüssigkeit immer noch recht aufwendig.

Eine Alternative besteht gegenwärtig in der Bestimmung der ionischen Dialysance. Das Grundprinzip dieser Messungen beruht auf der Tatsache, dass Harnstoff und kleine Ionen wie $Na^+$ etc. ein nahezu identisches Diffusionsverhalten haben. Die Konzentration dieser Ionen lässt sich in der Dialysierflüssigkeit leicht mit Hilfe von Messungen der elektrischen Leitfähigkeit bestimmen, welche mit relativ einfach aufgebauten Messzellen ermittelt werden kann. Anstelle der Harnstoff-Clearance wird daher zunächst die Ionen-Dialysance bestimmt. Diese kann dann - aufgrund des gleichen zu erwartenden Diffusionsverhaltens - gleich der Harnstoff-Clearance gesetzt werden.

Da die Clearance bei der Hämodialyse nur einen Spezialfall der Dialysance für den Fall darstellt, dass die betreffende Substanz nicht in der Dialysierflüssigkeit vorhanden ist, soll sie im Folgenden synonym mit dem Begriff Dialysance mitumfasst werden.

Im Stand der Technik finden sich diverse Veröffentlichungen zur Berechnung der Dialysance (z.B. J. Sargent und F. Gotch, in: Replacement of Renal Functions by Dialysis, 4. Auflage, herausgegeben von C. Jacobs et al., Kluwer, Dordrecht, 1996, S. 39ff). Ohne Ultrafiltration läßt sie sich in der sogenannten dialysatseitigen Form in der folgenden Form ausdrücken:

$$D = Qd \frac{Cdo - Cdi}{\alpha Cbi - Cdi} \qquad (1),$$

wobei

Qd:    Dialysierflüssigkeitsfluß,
Cdo:   Konzentration des betrachteten Stoffes in der abgeführten Dialysierflüssigkeit,
Cdi:   Konzentration des betrachteten Stoffes in der zugeführten Dialysierflüssigkeit,
Cbi:   Konzentration des betrachteten Stoffes im in den extrakorporalen Kreislauf einströmenden Blut (wobei nur der Volumenanteil zu betrachten ist, in dem dieser Stoff effektiv gelöst ist),
$\alpha$:    Gibbs-Donnan-Faktor.

Der Gibbs-Donnan Faktor berücksichtigt, dass auf der Blutseite geladene Ionen wie Na⁺ teilweise an entgegengesetzt geladene, nicht dialysatorgängige Proteine gebunden werden. Dieser Effekt hat zur Folge, dass sich im diffusiven Gleichgewicht (bei verschwindenden Flüssen) im Blutplasma eine etwas höhere IonenKonzentration als in der Dialysierflüssigkeit einstellen würde, da ein elektrisches Feld der Diffusion entgegenwirkt. Für den in der Praxis besonders relevanten Fall der Natriumionen im Blutplasma liegt $\alpha$ bei ca. 0,95. Sollte es die Genauigkeit nicht erfordern, kann dieser Faktor auch vernachlässigt werden.
In der Gleichung 1 können alle Größen außer Cbi leicht gemessen werden. Dazu genügt es, zwei Leitfähigkeitsmesszellen im Dialysierflüssigkeitskreislauf anzuordnen, die jeweils die Leitfähigkeiten am Eingang und Ausgang des Dialysators bestimmen. Letztere lassen sich leicht in die Konzentrationen Cdi und Cdo umrechnen. Falls die Konzentration Cdi auch vorgegeben und daher bekannt ist, weil z.B. genau definierte Flüssigkeiten verwendet werden, kann sich die Messung von Cdi sogar erübrigen. Der Dialysierflüssigkeitsfluss Qd wird meist durch die Hämodialysemaschine vorgegeben und ist daher ebenfalls bekannt. Andernfalls können natürlich zusätzlich entsprechende Sensoren vorgesehen sein.
Aus praktischen Gründen sind Leitfähigkeitsmessungen auf der Blutseite aber problematisch. Es ist jedoch möglich, durch eine Änderung der Konzentration Cdi den Term Cbi zu eliminieren. Dies kann z.B. in Form einer Konzentrationsstufe oder eines Bolus geschehen. Ersteres ist in der DE 39 38 662 A1 beschrieben, letzteres in der DE 197 47 360 A1 oder WO 00/02604 A1. Beide Möglichkeiten sollen im folgenden als Alternativen für eine Änderung der Konzentration in einer frischen Flüssigkeit gelten, die für die Blutbehandlung benötigt wird. Die Dialysance kann dann folgendermaßen bestimmt werden:

$$D = Qd(1 - \frac{Cdo2 - Cdo1}{Cdi2 - Cdi1}) = Qd(1 - \frac{\Delta Cdo}{\Delta Cdi}) \qquad (2),$$

wobei

Cdi1,2:   Cdi vor und nach (Stufe) bzw. außerhalb und während (Bolus) der Änderung
Cdo1,2:   Cdo vor und nach (Stufe) bzw. außerhalb und während (Bolus) der Änderung.

Im Falle einer Stufenänderung stellen $\Delta$Cdi bzw. $\Delta$Cdo einfache Differenzen dar, im Falle der Bolusmethode wird darunter die über den Bolus integrierte Änderung relativ zu einem Basisniveau verstanden.
Mit Hilfe von D kann nun auch Cbi mit Gleichung (1) bestimmt werden. Dabei wäre als äquivalent anzusehen, zunächst Cbi als zu bestimmenden Parameter aus einer zu Gleichung (2) entsprechenden Gleichung zu bestimmen, die aus Gleichung (1) hervorgeht, wenn D eliminiert wird.
Es sind weitere Verfahren im Stand der Technik wie die WO 98/32476 A1 oder EP 0 658 352 A1 bekannt, die nicht explizit die Gleichung (2) zur Bestimmung von D einsetzen, aber letztendlich immer auf dem Prinzip beruhen, eine Änderung einer physikalischen-chemischen Eigenschaft Cdi herbeizuführen und die entsprechende Änderung Cdo festzuhalten, um zu einer Aussage über die physikalisch-chemische Eigenschaft Cbi auf der Blutseite oder die Blutreinigungsleistung D zu gestatten. In EP 0 658 352 A1 oder US 5,567,320, die einer gemeinsamen Patentfamilie angehören, wird die Harnstoffclearance von der Dialysance D für Natrium abgeleitet.

Für die Beschreibung der Blutreinigungsleistung eines Blutreinigungselementes wie eines Dialysators wird gelegentlich auch der Massenaustausch- oder Filterkoeffizient k0A herangezogen, der zur Dialysance D in einer festen Beziehung steht. Der Koeffizient k0A wird allein durch den betrachteten Stoff und die verwendete Dialysatormembran bestimmt, nicht jedoch durch Behandlungsparameter wie Blut-, Dialysierflüssigkeit- oder Ultrafiltrationsfluss. Er ist das Produkt aus dem membranabhängigen Parameter k0 und der Gesamtfläche der Membran A. Dabei entspricht k0 dem Diffusionsstrom des betrachteten Stoffes pro Flächeneinheit der Membran, dividiert durch das Konzentrationsgefälle an der Membran. KOA kann als maximal mögliche Dialysance im Idealfall rein diffusiven Transports bei unendlich großen Dialysierflüssigkeit- und Blutflüssen interpretiert werden.

Der Koeffizient k0A für einen Stoff kann anhand der Messung der Dialysance D nach Gleichung (3) bestimmt werden:

$$k0A = \frac{QbQd}{Qd - Qb} \ln \frac{Qb(D - Qd)}{Qd(D - Qb)} \qquad (3).$$

Im Stand der Technik werden dabei ausnahmslos Verfahren angegeben, die eine Bestimmung während einer Hämodialysebehandlung erlauben. Zwar finden sich dort auch - zum Teil unterschiedliche - Angaben, wie der während einer Hämodialysebehandlung dem Blut entzogene Ultrafiltrationsfluss Qf in den Gleichungen (1) oder (2) berücksichtigt werden kann. Beispielhaft sei hier die EP 1 062 960 A2 genannt, wonach Qd durch die Summe der Flüsse Qd und Qf ersetzt wird. Bei einer Hämodialysebehandlung ist jedoch der Ultrafiltrationsfluss Qf sehr klein im Vergleich zum Dialysierflüssigkeitsfluss Qd und auch zum Blutfluss Qb, d.h. es handelt sich um einen verhältnismäßig kleinen Störeffekt. So liegen z.B. typische Werte bei Qf = 15 ml/min, Qd = 500 ml/min und Qb = 300 ml/min.

Für den Blutfluss Qb in Gleichung (3) gelten ähnliche Einschränkungen wie für die Konzentration Cbi in Gleichung (1). In Gleichung (3) muss zum Teil nur der Volumenanteil des Blutes betrachtet werden, in dem der betrachtete Stoff effektiv gelöst ist. Je nach Stoff kann dies z.B. der Blutwasseranteil ohne oder mit Blutzellen sein. Dem Fachmann sind dabei die Wege hinreichend bekannt, den bezüglich des Vollblutflusses anteiligen Fluss auf der Basis von durchschnittlichen, angenommen oder gemessenen Daten über die Blutzusammensetzung (Hämatokrit, Proteine, etc.) abzuleiten (z.B. J. Sargent und F. Gotch, in: Replacement of Renal Functions by Dialysis, 4. Auflage, herausgegeben von C. Jacobs et al., Kluwer, Dordrecht, 1996, S. 41ff), so dass an dieser Stelle von einer näheren Erläuterung abgesehen wird.

Bei einer Nierenersatzbehandlung ist aber die Kenntnis der Leistungsfähigkeit des Blutreinigungselementes genauso von Interesse, wenn es sich um eine Hämofiltrationsbehandlung handelt - sei es allein oder in Kombination mit einer Hämodialysebehandlung in Form einer Hämodiafiltrationsbehandlung.

Wie in der vorangemeldeten deutschen Patentanmeldung 10212247.4 beschrieben wird, lassen sich die für die Hämodialyse entwickelten Verfahren auf die Hämofiltration und die Hämodiafiltration übertragen, wenn der Dialysierflüssigkeitsfluss Qd den Substitutionsflüssigkeitsfluss enthält und die Konzentration für die frische Dialysierflüssigkeit gleich der Konzentration für die Substitutionsflüssigkeit ist. In diesem Fall ist der Dialysierflüssigkeitsfluss Qd in den Gleichungen (1) und (2) der Summe des in die erste Kammer des Hämodialysators fliessendes Flusses an Dialysierflüssigkeit, dem Fluss Qs der Substitutionsflüssigkeit und dem insgesamt dem Blut zu entziehenden Ultrafiltrationsfluss Qf zu setzen.

Mit den bisher genannten Verfahren ist es - wie oben ausgeführt - möglich, die Konzentration Cbi eines ersten Stoffes im der Blutreinigungseinheit zuströmenden Blut und/oder die Blutreinigungsleistung der Blutreinigungseinheit aufgrund von Konzentrationsmessungen in der Dialysierflüssigkeit zu bestimmen, wobei allerdings die Konzentration des ersten Stoffes in der Dialysierflüssigkeit während des Verfahrens geändert werden muss. Dies erfordert eine gewisse Mindestmesszeit für die entsprechende Einstellung oder Veränderung der Konzentration. Besonders nachteilig ist, dass mit diesen Methoden keine Stoffe zugänglich sind, die in der frischen Dialysierflüssigkeit im Allgemeinen nicht vorkommen (wie z.B. Kreatinin oder Phosphat) oder deren Variation im Sinne der Patientenverträglichkeit kritisch sein kann (wie z.B. Kalium).

Es sind andere Verfahren wie in der US 6,126,831 beschrieben bekannt, bei denen zur dialysatseitigen Messung von Blutbestandteilen der Dialysierflüssigkeitsfluss so verlangsamt oder sogar angehalten wird, dass die sich die Konzentration beider Flüssigkeiten so angleicht, dass die Konzentration in der Dialysierflüssigkeit direkt der Konzentration Cbi im Blut entspricht. Derartige Verfahren sind ebenfalls zeitaufwendig und gehen mit einem direkten Eingriff in die Blutbehandlung einher.

Der Erfindung liegt daher die Aufgabe zugrunde, eine Vorrichtung bereitzustellen, die ohne zusätzlichen Eingriff in die mit einem Blutreinigungselement erfolgende Blutbehandlung eine Bestimmung einer weiteren, verschiedenen Blutreinigungsleistung des Blutreinigungselementes bezogen auf einen weiteren Stoff erlaubt und damit die Möglichkeit eröffnet, auch die Blutkonzentration dieses weiteren Stoffes zu bestimmen.

Die Lösung gelingt durch die Merkmale des Anspruchs 1. Vorteilhafte Ausführungsformen sind Gegenstand der Unteransprüche.

Die Erfindung basiert auf der Beobachtung, dass heutige Hämodialysevorrichtungen oft über die Möglichkeit verfügen, in bewährter Art und Weise die Blutreinigungsleistung des Blutreinigungselementes - hier die Dialysance des Dialysators - bezogen auf einen ersten Stoff zu bestimmen. Dabei kann wie vorher beschrieben die Natriumionen-Dialysance mit Hilfe einer Veränderung der Konzentration in der frischen Dialysierflüssigkeit bestimmt werden. In diesem Fall ist es nun möglich, die zur ersten Blutreinigungsleistung verschiedene zweite Blutreinigungsleistung für einen zweiten Stoff zu bestimmen, ohne dass ein weiteres Messverfahren notwendig ist. Vielmehr kann aufgrund von in einer Auswerteeinheit hinterlegten Beziehungen zwischen den beiden Blutreinigungsleistungen, die über eine pure Identitätszuweisung bei gleichen Blutreinigungsleistungen wie im Fall von Natriumionen und Harnstoff hinausgehen, die zweite Blutreinigungsleistung direkt bestimmt werden. Diese Beziehung, die in Laborversuchen vorab ermittelt werden kann, hängt nur von der Art des verwendeten Blutreinigungselementes ab.

Die Erfindung weist dabei den Vorteil auf, dass durch die vorher erfolgte Messung der Blutreinigungsleistung für einen ersten Stoff eine individuelle Anpassung der Ist-Werte der Blutreinigungsleistung für einen zweiten Stoff ermöglicht wird, die der Veränderung der Blutreinigungsleistung eines bestimmten Blutreinigungselementes z.B. während einer Blutbehandlung ausreichend Rechnung trägt. Insofern geht die Erfindung über das bloße Berechnen der Blutreinigungsleistung für verschiedene Molekülgrößen durch Membrankenndaten hinaus.

Eine wichtige Fortbildung der Erfindung besteht darin, dass mit Hilfe eines Sensors zur Messung der Konzentration des zweiten Stoffes in der verbrauchten Dialysierflüssigkeit und - falls die Konzentration dieses Stoffes in der frischen Dialysierflüssigkeit nicht bekannt ist - eines entsprechenden Sensors für die frische Dialysierflüssigkeit, die Konzentration dieses Stoffes im dem Blutreinigungselement zuströmenden Blut mit Hilfe der vorher bestimmten Blutreinigungsleistung bestimmt werden kann, ohne dass ein Eingriff in die Dialysierflüssigkeitskonzentration oder die Fördergeschwindigkeiten der einzelnen Flüssigkeiten notwendig ist. Dies ist ohne Einschränkung für alle Stoffe möglich, deren Konzentration in der Dialysierflüssigkeit messtechnisch festgestellt werden kann - unabhängig von ihrem Vorhandensein in der frischen Dialysierflüssigkeit oder einer eingeschränkten Möglichkeit der Variation.

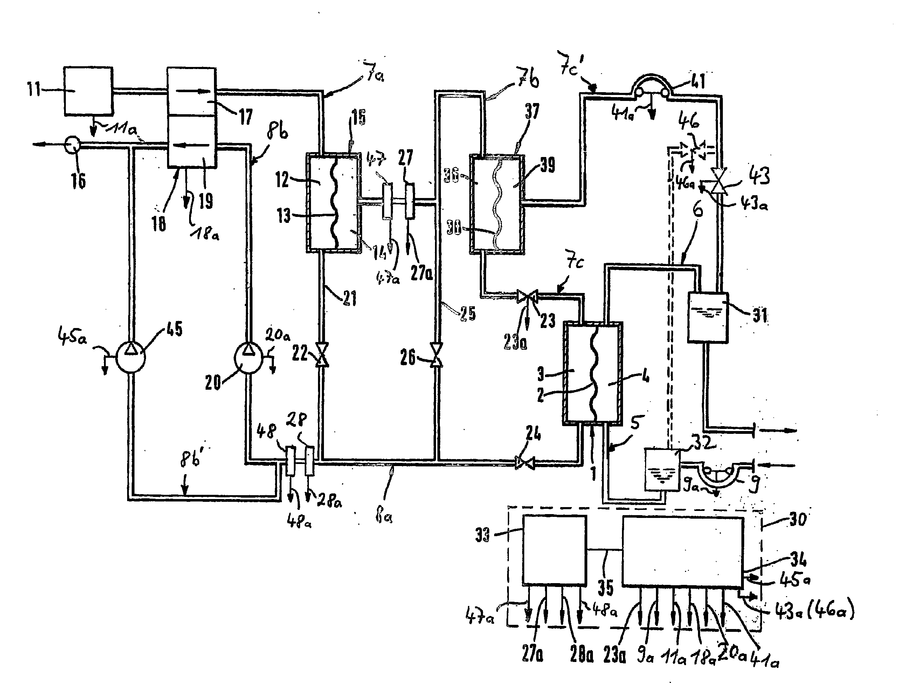

Die Erfindung sowie eine beispielhafte Ausführungsform einer erfindungsgemäßen Hämodiafiltrationsvorrichtung werden anschließend anhand der Zeichnung näher erläutert. Die Zeichnung zeigt dabei eine schematisierte Darstellung dieser Ausführungsform.

Kernstück der Hämodiafiltrationsvorrichtung ist der Hämodialysator 1. Der Hämodialysator 1 ist durch eine semipermeable Membran 2 in zwei Kammern 3 und 4 eingeteilt, von denen die erste Kammer 3 Teil eines Dialysierflüssigkeitskreislaufs und die zweite Kammer 4 Teil eines extrakorporalen Blutkreislaufs sind.

Der extrakorporale Blutkreislauf umfasst neben anderen, nicht näher gezeigten gebräuchlichen Komponenten eine Blutzuführleitung 5 mit einer Blutförderpumpe 9 und einem arteriellen Blasenfänger 32 zum Zuführen von Blut von einem Patienten zur Kammer 4 sowie eine Blutabführleitung 6 mit einem venösen Blasenfänger 31 zur Rückführung des Blutes zum Patienten.

Der Dialysierflüssigkeitskreislauf beinhaltet eine in Abschnitte 8a, 8b eingeteilte Dialysierflüssigkeitsabführleitung, von der eine Ultrafiltratabführleitung 8b' abzweigt. Der Abschnitt 8a führt aus der ersten Kammer 3 heraus, wobei ein Ventil 24 zu Absperrung dieser Ausgangsleitung des Hämodialysators vorgesehen ist. Am Ende des Abschnitts 8a ist ein als Leitfähigkeitsmesszelle 28 zur Erfassung der elektrischen Leitfähigkeit ausgebildeter erster abstromiger Sensor vorgesehen, mit der die Ionenkonzentration bzw. vorwiegend die Natriumkonzentration C1do auf bekannte Art und Weise ermittelt werden kann. Hierzu ist die Messzelle 28 mit einer zentralen Auswerte- und Steuereinheit 30 über eine Datenleitung 28a verbunden.

Im Abschnitt 8b ist die Dialysierflüssigkeitspumpe 20 eingebaut, an die keine besonderen Genauigkeitsanforderungen gestellt werden. Sie muss lediglich eine ausreichende Förderkapazität bereitstellen, damit die erste Bilanzkammerhälfte 19 einer Bilanzkammer 18, die in den Abschnitt 8b geschaltet ist, in vorgegebenen Zeiten gefüllt werden kann. Die Bilanzkammer 18 dient der Gewährleistung, dass der Abschnitt 8b nur von einem Teil des abgeführten Dialysierflüssigkeitsflusses durchflossen wird, der dem der Hämodiafiltrationsvorrichtung zugeführten Flüssigkeitsfluss entspricht (Substitutionsflüssigkeit mit Fluss Qs und frische Dialysierflüssigkeit mit Fluss Qd). Die Bilanzkammer 18 besteht dabei zweckmäßigerweise aus zwei parallel geschalteten Bilanzkammern, damit ein praktisch konstanter Fluss gewährleistet werden kann. Der Einfachheit halber wurde auf die Darstellung der zweiten Bilanzkammer sowie der diversen Eingangs- und Ausgangsventile in der Zeichnung verzichtet.

In dem Abschnitt 8b' ist eine als volumetrische, vorzugsweise als Membranpumpe ausgebildete Förderpumpe 45 vorgesehen. Mit dieser Pumpe wird der zu entfernende Ultratfiltratfluss Qf gefördert, der dem Patienten insgesamt entzogen werden soll. Die Bilanzkammer 18 sowie die Pumpen 20 und 45 sind mit entsprechenden Steuerleitungen 18a, 20a und 45a mit der Auswerte- und Steuereinheit 30 verbunden.

Die Abschnitte 8b und 8b' münden schließlich in einen Abfluss 16, wobei unerheblich ist, ob sich die beiden Abschnitte noch in der Vorrichtung wie gezeigt vereinigen oder nicht.

Frische Substitutions- und/oder Dialysierflüssigkeit wird von einer Flüssigkeitsquelle 11 bereitgestellt, die Teil eines Dialysierflüssigkeitsaufbereitungssystems ist. Zur Ausgestaltung der Flüssigkeitsquelle stehen dem Fachmann unterschiedliche Alternativen zur Verfügung. Neben einer Bereitstellung der fertigen Lösung in Beuteln ist dies insbesondere die Aufbereitung der Flüssigkeit in der Hämodiafiltrationsvorrichtung selbst aus Wasser und Konzentrat. Die Vorrichtung enthält hierfür diverse Mess- und Steuerelemente, auf deren Erläuterung an dieser Stelle abgesehen wird und die hinlänglich bekannt sind.

Der Dialysierflüssigkeitskreislauf umfasst ferner die folgenden Komponenten: Die frische Dialysierflüssigkeit fließt von der Flüssigkeitsquelle 11 durch einen ersten Abschnitt 7a einer Dialysierflüssigkeitszuführleitung, an den sich die Abschnitte 7b und 7c anschließen. In den Abschnitt 7a ist die zweite Bilanzkammerhälfte 17 der Bilanzkammer 18 geschaltet. Er mündet schließlich in die erste Kammer 12 eines durch eine semipermable Membran 13 in zwei Kammern 12 und 14 geteilten ersten Sterilfilters 15. Die Flüssigkeit verläßt nach Passage der Membran 13 die zweite Kammer 14 des ersten Sterilfilters über den Abschnitt 7b der Dialysierflüssigkeitszuführleitung, die zur ersten Kammer 36 eines durch eine semipermable Membran 38 in zwei Kammern 36 und 39 geteilten zweiten Sterilfilters 37 führt. In den Abschnitt 7b ist ein dem ersten abstromigen Sensor 28 entsprechender erster aufstromiger Sensor 27 zur Erfassung der elektrischen Leitfähigkeit der diesen Sensor durchfließenden Flüssigkeit vorgesehen, der wiederum mit einer Datenleitung 27a mit der Auswerte- und Steuereinheit 30 verbunden ist.

Die die Membran 38 passierende Substitutionsflüssigkeit verläßt die zweite Kammer 39 des Sterilfilters 37 über die Substitutionsflüssigkeitsleitung 7c'. In diesem Abschnitt ist eine Förderpumpe 41 zur Förderung des Substitutionsflüssigkeitsflusses Qs vorgesehen. Bevor die Substitutionsleitung 7c' in den venösen Blasenfänger 31 mündet (Postdilution), ist ein Absperrventil 43 vorsehen. Alternativ oder zusätzlich kann vorgesehen sein (gestrichelt gezeichnet), die Substitutionsflüssigkeitsleitung 7c' in den arteriellen Blasenfänger münden zu lassen (Prädilution). In diesem Abschnitt wäre dann ein weiteres Absperrventil 46 vorgesehen.

Aus der ersten Kammer 36 des zweiten Sterilfilters 37 führt ein Abschnitt 7c der Dialysierflüssigkeitsleitung zur ersten Kammer 3 des Hämodialysators 1. Der Abschnitt 7c ist durch ein Absperrventil 23 verschließbar, das über die Steuerleitung 23a mit der Auswerte- und Steuereinheit 30 verbunden ist. Mit diesem Ventil kann somit gesteuert werden, ob eine Blutbehandlung als reine Hämofiltrationsbehandlung (Ventil geschlossen) oder als Teil einer Hämodiafiltrationsbehandlung durchgeführt werden soll (Ventil geöffnet). Es ist auch möglich, während einer Behandlung den Behandlungsmodus zu wechseln. Des Weiteren kann durch das Anhalten der Pumpe 41 und das Schließen der Ventile 43 und 46 jederzeit eine reine Hämodialysebehandlung durchgeführt werden.

Mit Hilfe der Ventile 43 und 46 (Steuerung über Leitungen 43a und 46a) kann zwischen Prä- und Postdilution gewechselt oder sogar beides gleichzeitig ermöglicht werden. Hierzu kann vorgesehen sein, die Ventile 43 und 46 zur Flusssteuerung einzusetzen oder durch eigene Fördermittel zu ergänzen/auszutauschen um die Aufteilung des Substitutionsflüssigkeitsflusses Qs zu erfassen.

Für hier nicht näher beschriebene Sicherheits- und Reinigungsfunktionen ist des Weiteren eine erste Bypassleitung 21 vorgesehen, die die erste Kammer 12 des ersten Sterilfilters 15 mit dem Abschnitt 8a der Dialysierflüssigkeitsabführleitung verbindet und welche durch ein Ventil 22 während des normalen Betriebs verschließbar ist. Gleiches gilt für eine zweite Bypassleitung 25, die von dem Abschnitt 7b der Dialysierflüssigkeitszuführleitung abzweigt und aufstromig ebenfalls in den Abschnitt 8a der Dialysierflüssigkeitsabführleitung mündet. Die zweite Bypassleitung ist durch ein Ventil 26 verschließbar.

Die Hämodiafiltrationsvorrichtung enthält ferner eine Auswerte- und Steuereinheit 30, die ihrerseits aus einer Auswerteeinheit 33 und einer Steuereinheit 34 besteht, die durch einen Datenleitung 35 miteinander verbunden sind. Die Steuereinheit ist über die Steuerleitungen 9a, 11a, 18a, 20a, 23a, 41 a, 43a, 45a und 46a mit den diversen Steuerelementen der Hämodiafiltrationsvorrichtung verbunden, um deren Betrieb steuern zu können. Dabei wurden nur die Steuerelemente/-leitungen erwähnt, die für das Verständnis der Erfindung erforderlich sind.

Die Auswerteeinheit ist über Datenleitungen mit einigen Sensoren verbunden. Im vorliegenden Fall sind dies im Besonderen die beiden Leitfähigkeitssensoren 27 und 28. Des Weiteren sind ein zweiter aufstromiger Sensor 47 und ein zweiter abstromiger Sensor 48 zur Erfassung der Konzentration eines zweiten Stoffes wie z.B. Kalium, Calcium, Phosphat, Kreatinin oder Glukose im Dialysierflüssigkeitskreislauf vorgesehen. Zur Ausgestaltung derartiger zweiter Sensoren 47 und 48 sind dem Fachmann verschiedenste, dem Zweck angepasste Ausführungsformen geläufig. Die Sensoren 47 und 48 sind über Datenleitungen 47a und 48a mit der Auswerteeinheit 33 verbunden. Der Einsatz eines zweiten aufstromigen Sensors kann sich in dem Fall erübrigen, in dem die Konzentration des zweiten Stoffes in der frischen Dialysierflüssigkeit bekannt ist. Dies kann insbesondere dann mit hoher Genauigkeit ausgenutzt werden, wenn der zweite Stoff in der frischen Dialysierflüssigkeit gar nicht enthalten ist, wie z.B. bei Körperausscheidungsprodukten wie Kreatinin.

Das Volumen einer Bilanzkammerfüllung ist sehr genau bekannt. Über die Frequenz der Bilanzkammertakte kann der Fluss Qs+Qd sehr genau bestimmt werden. Die Pumpe 45 ist volumetrisch und kann damit ebenfalls genutzt werden, um - wie hier als Membranpumpe über die Frequenz der Pumphübe und das bekannte Hubvolumen - den Fluss Qf zu bestimmen. Dies beseitigt Ungenauigkeiten, die z.B. durch eine als Rollenpumpe gestaltete Substitutionsflüssigkeitspumpe 41 entstehen, deren Fördermenge aufgrund von Toleranzschwankungen des Pumpschlauchsegments und auch durch Ladedruckschwankungen in einem gewissen Bereich schwanken kann.

Die erfindungsgemäße Vorrichtung ist geeignet, die folgenden Verfahrensschritte durchzuführen. Dabei sei der Einfachheit halber zunächst angenommen, dass während der Erfassung der Messwerte eine reine Hämodialysebehandlung ohne Ultrafiltration durchgeführt wird, also Qs=Qf=0 ist.

Die Flüssigkeitsquelle 11 wird so angesteuert, dass sie Dialysierflüssigkeit mit einer Natriumkonzentration C1di1 bereitstellt. Diese Konzentration wird über den ersten aufstromigen Sensor 27 aufgezeichnet und an die Auswerteeinheit 33 übermittelt. An den Fördereinrichtungen/Pumpen 9, 18, 20, 41 und 45 werden die Flüssigkeitsflüsse Qb und Qd eingestellt sowie die Ventile 23, 43 und 46 für die Betriebsart der Hämodialyse geöffnet bzw. geschlossen. Die Werte für Qb und Qd werden außerdem von der Steuereinheit 34 an die Auswerteeinheit 33 übermittelt. Die Natriumkonzentrationswerte C1do1 werden durch den ersten abstromigen Sensor 28 aufgezeichnet und an die Auswerteeinheit 33 übermittelt.

Zu einem Zeitpunkt, an dem der Steuerablauf dies automatisiert vorsieht oder dies aus einem anderen Grund (z.B. manuell) veranlasst wurde, führt die Flüssigkeitsquelle 11 auf Anweisung der Steuereinheit 34 eine Änderung der Natriumkonzentration der Dialysierflüssigkeit z.B. in Bolusform durch, d.h. die Natriumkonzentration wird kurzzeitig verändert und nimmt danach wieder den Ausgangswert an. Die entsprechenden Konzentrationen C1di2 und C1do2 werden aufgezeichnet und an die Auswerteeinheit 33 übermittelt. Nach dem Abklingen des Bolus bestimmt die Auswerteeinheit 33 die Ionen-Dialysance bzw. Natriumionen-Dialysance D1 der Hämodiafiltrationsvorrichtung als Blutreinigungsleistung L1 des Blutreinigungselementes 1 für einen ersten Stoff in der bekannten Art und Weise mit Hilfe von Gleichung (2). Dieser Wert kann dann über eine nicht gezeigte Anzeigeeinheit, die meist sowie Teil derartiger Blutbehandlungsvorrichtungen ist, angezeigt werden. Die gemessene Wert Dialysance D1 wird im Folgenden als effektive Dialysance D1eff bezeichnet, um sie von der aufgrund der Kenntnis des Membranmaterials zu erwartenden, theoretischen Dialysance D1th abgrenzen zu können.

Zur Bestimmung der Blutreinigungsleistung L2 des Blutreinigungselementes 1 für einen zweiten Stoff ist die Auswerteeinheit 33 erfindungsgemäß geeignet, eins der beiden im folgenden beschriebenen Verfahren anzuwenden. Bei beiden Verfahren wird von der Auswerteeinheit von vorher ermittelten Massenaustauschkoeffizienten k0A1,2 für die beiden zu betrachtenden Stoffe ausgegangen, die in einer festgelegten Beziehung zueinander stehen:

$$k0A2 = f \cdot k0A1 \tag{4}.$$

Für einen von der Anmelderin unter der Bezeichnung F60 vertriebenen Dialysefilter konnte z.B. für Harnstoff (beziehungsweise Natrium) als ersten Stoff und für Kalium als zweiten Stoff Werte von k0A1=734.7ml/min sowie f=1,08 gefunden werden.

Entweder sind diese Werte oder die Werte von k0A1 und k0A2 in der Auswerteeinheit 33 hinterlegt.

Mit Hilfe der bekannten Werte kann die Auswerteeinheit 33 durch Auflösung der Gleichung (3) die entsprechenden theoretischen Dialysance-Werte D1th und D2th bestimmen, da die Werte für den Blutfluss Qb und den Dialysierflüssigkeitsfluss Qd ebenfalls in der Auswerteeinheit 33 abgelegt sind. Mit Hilfe des gemessenen, effektiven Dialysance-Wertes D1eff für Natrium kann nun der effektive Dialysance-Wert D2eff für Kalium mit Gleichung (5) ermittelt werden:

$$D2eff = D1eff \frac{D2th}{D1th} \tag{5}.$$

Andererseits ist es auch möglich, dass die Auswerteeinheit 33 zunächst anhand der gemessenen Dialysance D1eff für Natrium den entsprechenden effektiven Massenaustauschkoeffizienten k0A1eff mit Hilfe von Gleichung (3) bestimmt. Der hinterlegte Werte für f wird dann dazu verwendet, mit Gleichung (4) den effektiven Massenaustauschkoeffizienten k0A2eff für Kalium zu bestimmen. Dieser wird dann wiederum verwendet, um mit Hilfe von Gleichung (3) die zu bestimmende effektive Dialysance D2eff für Kalium zu bestimmen. Im Gegensatz zur ersten Methode braucht in diesem Fall nur der Faktor f und nicht zusätzlich der Wert für k0A1th hinterlegt zu werden.

Die hinterlegten Werte für k0A können für eine Serie von Dialysatoren, die sich nur in der aktiven Membranfläche A unterscheiden, aber die gleiche Membranart aufweisen, dahingehend gespeichert werden, dass nur ein membranspezifischer Wert (wie k0) hinterlegt zu werden braucht, während sich die anderen Werte durch die Proportionalität zu A entsprechend berechnen lassen. Bei der zweiten Methode ist dies nicht erforderlich, da der Faktor f unabhängig von der aktiven Membranfläche A ist.

Es liegt auf der Hand, dass die Berechnungen für jeden zweiten Stoff durchgeführt werden können, für den entsprechende Daten nach Gleichung (4) vorliegen. So gilt z.B. für die F60-Membran f=0,52 für Glukose, f=0,71 für Kreatinin und f=0,66 für Phosphat.

Nachdem die Auswerteeinheit 33 die Dialysance D2eff als Blutreinigungsleistung des Blutreinigungselementes 1 bezogen auf einen zweiten Stoff bestimmt hat, kann dieser auf einer Anzeigeeinheit dem Benutzer ebenfalls zur Kenntis gebracht werden.

In einer besonders vorteilhaften Ausführungsform der Erfindung wird der ermittelte Wert von D2eff verwendet, um die Konzentration C2bi des zweiten Stoffes in der Blutzuführleitung 5 zu bestimmen. Hierzu werden die Messwerte der zweiten aufstromigen und abstromigen Sensoren 47 und 48, die die Konzentrationen C2di und C2do des zweiten Stoffes in der frischen und verbrauchten Dialysierflüssigkeit feststellen, durch die Auswerteeinheit 33 erfasst. Hierfür ist keinerlei Eingriff in den Behandlungsablauf notwendig. Die Auswerteeinheit bestimmt dann C2bi durch Auflösung von Gleichung (1) nach Cbi.

Die beiden beschriebenen Verfahren zur Anwendung der Gleichung (4) führen bei der Anwendung auf "ideale" Systeme zu gleichen numerischen Ergebnissen. In der praktischen Anwendung kommt es jedoch zu kleineren Abweichungen, deren Ursachen im Folgenden näher erläutert werden.

Eine der Hauptursachen für die Abweichungen liegt in dem Umstand, dass in einem realen Dialysesystem eine Rezirkulation von gereinigtem Blut auftritt, wodurch die theoretisch erreichbare Dialysance Dth vermindert wird. Per Messung ist nur die entsprechend verminderte effektive Dialysance Deff feststellbar. Die Rezirkulation kann dabei in dem Patientengefäß - meist einer arterio-venösen Fistel - auftreten, dem das Blut entnommen und wieder zurückgegeben wird. In diesem Fall kann gereinigtes Blut unmittelbar zum Dialysator 1 zurückkehren. Diese sogenannte Fistelrezirkulation kann jedoch durch eine geeignete Wahl des Blutflusses Qb weitgehend vermieden werden, solange der Blutfluss Qb kleiner als dem der Fistel zufließenden Blutfluss ist. Ein Teil des gereinigten Blutes kehrt jedoch als sogenannte kardiopulmonäre Rezirkulation direkt über das Gefäßsystem des Patienten zur Fistel zurück, ohne einen Stoffwechsel durchlaufen zu haben. Dieser Teil der Rezirkulation tritt inhärent auf und kann nicht vermieden werden, auch wenn es sich um keinen dominierenden Effekt handelt.

Der Einfluss der Rezirkulation auf die Dialysance oder Clearance ist unter anderem von H.D. Polaschegg und N.W. Levin (in "Replacement of Renal Functions by Dialysis", 4. Auflage, herausgegeben von C. Jacobs et al. , Kluwer, Dordrecht, 1996, S. 371) beschrieben worden. Danach gibt es den folgenden Zusammenhang zwischen der durch die Rezirkulation R verminderten effektiven Dialysance Deff und der entsprechenden Dialysance Dth ohne Rezirkulation für den gleichen Dialysator und die gleichen Flussverhältnisse:

$$Deff = Dth \frac{1-R}{1-R(1-\frac{Dth}{Qb})} \qquad (6),$$

wobei R den Anteil des rezirkulierten Blutes zwischen 0 und 1 an dem Blutfluss Qb angibt. Falls die Rezirkulation R bekannt ist (durch andere Messverfahren), kann Gleichung (6) für die Verbesserung der Genauigkeit von D2eff berücksichtigt werden.

Weitere Unterschiede zwischen den beiden Rechenmethoden werden dadurch verursacht, dass die verwendeten Parameter wie Flüssigkeitsflüsse oder auch die Werte für k0A und/oder f nur innerhalb gewisser Fehlergrenzen bekannt sind. Dies führt durch den Eingang des Ist-Wertes D1 eff für die Dialysance für den ersten Stoff zu unterschiedlichen Folgefehlern für D2eff, deren Einfluss jedoch begrenzt ist und durch Kalibrierungsmessungen im Labor vorab untersucht werden kann.

Die Erfindung kann nicht nur bei der reinen Hämodialyse, sondern auch im Fall einer nicht ausgeschalteten Ultrafiltration (Qf>0) und/oder der Hämodiafiltration (Qs>0) angewendet werden. Wie in der deutschen Patentanmeldung 10212247.4 ausgeführt werden dazu der Auswerteeinheit 33 über die Leitung 35 nicht nur die Werte für Qd und Qb, sondern auch für Qf und für Qs übermittelt. Die Auswerteeinheit 33 kann dann den diffusiven Teil der Dialysance anhand Gleichung (7) bestimmen:

$$Ddiff = \frac{Qb + \kappa Qs}{Qb - Qf - (1 - \kappa)Qs}\left(\frac{Qb + \kappa Qs}{Qb}D - Qf - Qs\right) \tag{7},$$

wobei $\kappa=1$ bei Prädilution und $\kappa=0$ bei Postdilution. Daraufhin kann der Membranaustauschkoeffizient k0A bestimmt werden, für den nur der diffusive Teil der Dialysance relevant ist:

$$k0A = \frac{(Qb + \kappa Qs)Qd}{Qd - Qb - \kappa Qs}\ln\frac{\dfrac{Ddiff}{Qd} - 1}{\dfrac{Ddiff}{Qb + \kappa Qs} - 1} \tag{8}.$$

Gleichung (8) entspricht einer Verallgemeinerung von Gleichung (3).

Die in der Zeichnung dargestellte Ausführungsform weist erste und zweite aufstromige Sensoren 27 und 47 zur Messung der Konzentrationen C1di des ersten Stoffes und C2di des zweiten Stoffes in der frischen Dialysierflüssigkeit auf. Alternativ zu diesen aufstromigen Sensoren können zur Messung der Konzentrationen in der frischen Dialysierflüssigkeit auch die abstromigen Sensoren 28 und 48 eingesetzt werden, wenn die frische Dialysierflüssigkeit unter Umgehung des Dialysators direkt zu den abstromigen Sensoren geleitet wird. Dies kann durch Öffnung der Bypass-Ventile 22 oder 26 geschehen.

Diese alternative Ausführungsform ist insbesondere für die Messung des zweiten Stoffes nicht mit besonderen Nachteilen verbunden. Da die Konzentration des zweiten Stoffes in der frischen Dialysierflüssigkeit in den meisten Fällen konstant bleibt, ist nur eine einzige Messung am Beginn der Behandlung erforderlich. Sollte sich diese Konzentration aufgrund einer gesteuerten Variation während einer Blutbehandlung dennoch ändern, kann die Messung jeweils durch eine kurze Schaltung in den Bypass aktualisiert werden. Falls bei der Blutbehandlungsvorrichtung eine solche Bypass-Schaltung aufgrund von anderen Verfahrensschritten periodisch durchgeführt wird (z.B. zur der Spülung des ersten Sterilfilters 15), kann die Messung des zweiten Stoffes ohne zusätzliche Verfahrensschritte gleichzeitig durchgeführt werden.

Die Erfindung ermöglicht damit eine einfache und unkomplizierte Bestimmung der Blutreinigungsleistung eines Blutreinigungselementes für einen zweiten Stoff, nachdem die davon abweichende Blutreinigungsleistung für einen ersten Stoff zuvor ermittelt wurde. Des Weiteren wird die Bestimmung der Blutkonzentration von Stoffen durch Messungen in der Dialysierflüssigkeit ermöglicht, die vorher aufgrund der schlechten Zugänglichkeit der aktuellen Blutreinigungsleistung des Blutreinigungselementes bezogen auf diese Stoffe nicht möglich war. Dies gestattet eine patientenverträglichere Blutbehandlung.

Durch die Bestimmung der Kaliumkonzentration kann Arrhytmien während der Dialyse besser vorgebeugt werden. Die Überwachung der Glukosekonzentration bietet insbesondere bei diabetischen Patienten einen wichtigen Aspekt zur Vermeidung von Komplikationen. Die Kenntnis der Calciumkonzentration im Blut ist besonders wichtig beim Einsatz von Citrat als Antikoagulanzmittel. In diesem Fall muss Calcium in die Blutabführleitung infundiert werden, damit das Citrat vor der Rückgabe des Blutes an den Patienten gebunden wird. Dabei ist darauf zu achten, dass die Calciumkonzentration als Folgeerscheinung nicht zu hoch wird. Die Kenntnis der Phosphatkonzentration stellt ebenfalls eine wichtige Information dar, da insbesondere bei Dialysepatienten die Gefahr besteht, dass ein zu hoher Phosphatspiegel zu Ablagerungen von Calciumphosphat im Gewebe führt.

Durch die erfindungsmäße Vorrichtung stehen die entsprechenden Messwerte unmittelbar während der Blutbehandlung zur Verfügung, ohne dass es der aufwendigen Analyse von Blutproben in einem Labor bedarf.

Bezugszeichenliste

1    Dialysator
2    semipermeable Membran des Dialysators
3    erste Kammer des Dialysators
4    zweite Kammer des Dialysators

5     Blutzuführleitung

6     Blutabführleitung

7a     erster Abschnitt der Dialysierflüssigkeitsleitung

7b     zweiter Abschnitt der Dialysierflüssigkeitleitung

7c     dritter Abschnitt der Dialysierflüssigkeitsleitung

7c'     Substitutionsflüssigkeitleitung

8a     erster Abschnitt der Dialysierflüssigkeitsabführleitung

8b     zweiter Abschnitt der Dialysierflüssigkeitsabführleitung

8b'     Ultrafiltratabführleitung für Flüssigkeitsentzug

9     Blutpumpe

11     Dialysier-/Substitutionsflüssigkeitsquelle

12     erste Kammer des ersten Sterilfilters

13     semipermeable Membran des ersten Sterilfilters

14     zweite Kammer des ersten Sterilfilters

15     erster Sterilfilter

16     Abfluß

17     zweite Bilanzkammerhälfte

18     Bilanzkammer

19     erste Bilanzkammerhälfte

20     Dialysierflüssigkeitumwälzpumpe

21     erste Bypass-Leitung

22     erstes Bypass-Ventil

23     Dialysierflüssigkeitszuführventil

24     Dialysierflüssigkeitsabführventil

25     zweite Bypass-Leitung

26     zweites Bypass-Ventil

27     erster aufstromiger Sensor zur Erfassung der Leitfähigkeit der frischen Dialysierflüssigkeit zur Feststellung der Natriumionenkonzentration

28     erster abstromiger Sensor zur Erfassung der Leitfähigkeit der verbrauchten Dialysierflüssigkeit zur Feststellung der Natriumionenkonzentration

30     Auswerte- und Steuereinheit

31     venöser Blasenfänger

32     arterieller Blasenfänger

33     Auswerteeinheit

34     Steuereinheit

35     Datenleitung zwischen Auswerte- und Steuereinheit

36     erste Kammer des ersten Sterilfilters

37     zweites Sterilfilter

38     semipermeable Membran des zweiten Sterilfilters

39     zweite Kammer des zweiten Sterilfilters

41     Substitutionsflüssigkeitspumpe

43     Postdilutionventil

45     Pumpe für Flüssigkeitsentzug

46     Prädilutionsventil

47     zweiter aufstromiger Sensor zur Erfassung der Konzentration eines zweiten Stoffes in der frischen Dialysierflüssigkeit

48     zweiter abstromiger Sensor zur Erfassung der Konzentration des zweiten Stoffes in der verbrauchten Dialysierflüssigkeit

**Patentansprüche**

1. Blutbehandlungsvorrichtung mit einem durch eine semipermeable Membran (2) in zwei Kammern geteilten Blutreinigungselement (1), deren erste Kammer (3) Teil eines Dialysierflüssigkeitskreislaufs und deren zweite Kammer (4) Teil eines extrakorporalen Blutkreislaufs ist,
mit einer Dialysierflüssigkeitszuführleitung zur Zuführung frischer Dialysierflüssigkeit zur ersten Kammer (3) und/oder in den Blutkreislauf,
mit einer Dialysierflüssigkeitsabführleitung zur Abführung verbrauchter Dialysierflüssigkeit aus der ersten Kammer (3),

mit einer Steuereinheit (34) zur Steuerung der Blutbehandlungvorrichtung,

mit einer Auswerteeinheit (33),

mit mindestens einem mit der Auswerteeinheit (33) verbundenen Sensor (27, 28) an mindestens einem des Blutkreislauf oder Dialysierflüssigkeitskreislauf zur Erfassung der Konzentration eines ersten Stoffes, der die semipermeable Membran (2) durchdringen kann,

wobei die Auswerteeinheit (33) geeignet ist, anhand der Messwerte des mindestens einen Sensors (27, 28) die Blutreinigungsleistung L1 des Blutreinigungselementes für den ersten Stoff zu bestimmen,

**dadurch gekennzeichnet,**

**dass** die Auswerteeinheit (33) weiterhin geeignet ist, die von der Blutreinigungsleistung L1 für den ersten Stoff verschiedene Blutreinigungsleistung L2 des Blutreinigungselementes für einen zweiten Stoff auf der Basis der Blutreinigungsleistung L1 für den ersten Stoff zu bestimmen und

**dass** der zweite Stoff Kalium, Glukose oder Calcium ist.

2. Blutbehandlungsvorrichtung nach Anspruch 1, **dadurch gekennzeichnet, dass** die Blutreinigungsleistung L die effektive Dialysance Deff ist.

3. Blutbehandlungsvorrichtung nach Anspruch 2, **dadurch gekennzeichnet, dass** die Auswerteeinheit (33) geeignet ist, aus der gemessenen Dialysance D1eff für den ersten Stoff einen effektiven Massenaustauschkoeffizienten k0A1eff abzuleiten, aus dem hinterlegten Verhältnis f zwischen dem theoretischen Massenaustauschkoeffizenten k0A2th des zweiten Stoffes zum theoretischen Massenaustauschkoeffizenten k0A1th des ersten Stoffes durch Multiplikation mit k0A1eff den effektiven Massenaustauschkoeffizienten k0A2eff für den zweiten Stoff zu bestimmen und aus k0A2eff die effektive Dialysance D2eff für den zweiten Stoff abzuleiten.

4. Blutbehandlungsvorrichtung nach Anspruch 2, **dadurch gekennzeichnet, dass** die Auswerteeinheit (33) geeignet ist, aus den hinterlegten theoretischen Massenaustauschkoeffizienten k0A1th für den ersten Stoff und k0A2th für den zweiten Stoff diesen entsprechende Werte für die theoretischen Dialysancen D1th und D2th abzuleiten und die effektive Dialysance D2eff für den zweiten Stoff aus der gemessenen Dialysance D1eff für den ersten Stoff multipliziert mit dem Verhältnis D2th zu D1th zu bestimmen.

5. Blutbehandlungvorrichtung nach einem der vorhergehenden Ansprüche, **dadurch gekennzeichnet, dass** der mindestens eine Sensor ein erster abstromigen Sensor (28) an der Dialysierflüssigkeitsabführleitung (8a) zur Messung der Konzentration des ersten Stoffes in der verbrauchten Dialysierflüssigkeit ist.

6. Blutbehandlungvorrichtung nach Anspruch 5, **dadurch gekennzeichnet, dass** sie ferner eine mit der Steuereinheit (34) verbundene Dialysierflüssigkeitsaufbereitungseinheit (11) umfasst.

7. Blutbehandlungsvorrichtung nach Anspruch 6, **dadurch gekennzeichnet, dass** die Auswerteeinheit (33) und die Steuereinheit (34) geeignet sind, die Bestimmung der Blutreinigungsleistung L1 für den ersten Stoffes durch folgendes Verfahren durchzuführen:

Hinterlegung der Konzentration C1di1 des ersten Stoffes in der frischen Dialysierflüssigkeit in der Auswerteeinheit (33),

Messung der Konzentration C1do1 des ersten Stoffes in der verbrauchten Dialysierflüssigkeit mit dem ersten abstromigen Sensor (28) und Hinterlegung von C1do1 in der Auswerteeinheit (33),

Veränderung der Konzentration C1di des ersten Stoffes in der frischen Dialysierflüssigkeit durch die Dialysierflüssigkeitsaufbereitungseinheit (11) auf Veranlassung der Steuereinheit (34),

Hinterlegung der veränderten Konzentration C1di2 des ersten Stoffes in der frischen Dialysierflüssigkeit in der Auswerteeinheit (33),

Messung der veränderten Konzentration C1do2 des ersten Stoffes in der verbrauchten Dialysierflüssigkeit mit dem ersten abstromigen Sensor (28) und Hinterlegung von C1do2 in der Auswerteeinheit (33) und

Bestimmung der Blutreinigungsleistung L1 auf der Basis der Konzentrationen C1di1, C1do1 und veränderten Konzentrationen Cldi2, C1do2 des ersten Stoffes in der frischen und verbrauchten Dialysierflüssigkeit durch die Auswerteeinheit (33).

8. Blutbehandlungsvorrichtung nach Anspruch 7, **dadurch gekennzeichnet, dass** die Dialysierflüssigkeitsaufbereitungseinheit (11) die Veränderung der Konzentration C1di stufen- oder bolusförmig durchführt.

9. Blutbehandlungsvorrichtung nach Anspruch 7, **dadurch gekennzeichnet, dass** sie ferner einen mit der Auswerteeinheit (33) verbundenen und an der Dialysierflüssigskeitszuführleitung (7b) gelegenen ersten aufstromigen Sensor (27) zur Messung der Konzentrationen C1di1 und C1di2 in der frischen Dialysierflüssigkeit umfasst.

10. Blutbehandlungsvorrichtung nach einem der vorhergehenden Ansprüche, **dadurch gekennzeichnet, dass** sie ferner einen mit der Auswerteeinheit (33) verbundenen und an der Dialysierflüssigkeitsabführleitung (8a) gelegenen zweiten abstromigen Sensor (48) zur Messung der Konzentration C2do des zweiten Stoffes in der verbrauchten Dialysierflüssigkeit umfasst.

11. Blutbehandlungsvorrichtung nach Anspruch 10, **dadurch gekennzeichnet, dass** die Auswerteeinheit (33) geeignet ist, auf der Basis der gemessenen Konzentration C2do des zweiten Stoffes in der verbrauchten Dialysierflüssigkeit und der hinterlegten Konzentration C2di des zweiten Stoffes in der frischen Dialysierflüssigkeit sowie der bestimmten Blutreinigungsleistung L2 des zweiten Stoffes die Konzentration C2bi des zweiten Stoffes in dem der zweiten Kammer (4) zuströmenden Blut zu bestimmen.

12. Blutbehandlungsvorrichtung nach einem der vorhergehenden Ansprüche, **dadurch gekennzeichnet, dass** der erste Stoff Natrium ist.

**Claims**

1. A blood treatment device having a blood purification element (1) which is divided into two chambers by a semipermeable membrane (2), the first chamber (3) of which is part of the dialysis fluid circulation and the second chamber (4) of which is part of an extracorporeal blood circulation,
   having a dialysis fluid supply line for supplying fresh dialysis fluid to the first chamber (3) and/or to the blood circulation,
   having a dialysis fluid removal line for removing spent dialysis fluid from the first chamber (3),
   having a control unit (34) for controlling the blood treatment device,
   having an evaluation unit (33),
   having at least one sensor (27, 28) connected to the evaluation unit (33) on at least one of the blood circulation or the dialysis fluid circulation for detecting the concentration of a first substance, which is able to penetrate through the semipermeable membrane (2),
   the evaluation unit (33) being suitable for determining the blood purification performance L1 of the blood purification element for the first substance on the basis of the measured values of the at least one sensor (27, 28),
   **characterized in that**
   the evaluation unit (33) is also suitable for determining the blood purification performance L2 of the blood purification element for a second substance, which is different from the blood purification performance L1 for the first substance, on the basis of the blood purification performance for the first substance, and
   the second substance is potassium, glucose or calcium.

2. The blood treatment device according to claim 1, **characterized in that** the blood purification performance is the effective dialysance Deff.

3. The blood treatment device according to claim 2, **characterized in that** the evaluation unit (33) is suitable for deriving an effective mass exchange coefficient k0A1 eff from the measured dialysance D1 eff for the first substance, determining the effective mass exchange coefficient k0A2eff for the second substance from the stored ratio f between the theoretical mass exchange coefficient k0A2th of the second substance in relation to the theoretical mass exchange coefficient k0A1th of the first substance by multiplication times k0A1eff, and deriving from k0A2eff the effective dialysance D2eff for the second substance.

4. The blood treatment device according to claim 2, **characterized in that** the evaluation unit (33) is suitable for deriving the values for the theoretical dialysances D1th and D2th from the stored theoretical mass exchange coefficients k0A1th for the first substance and k0A2th for the second substance corresponding thereto, and determining the effective dialysance D2eff for the second substance from the measured dialysance D1 eff for the first substance multiplied times the ratio of D2th to D1th.

5. The blood treatment device according to any one of the preceding claims, **characterized in that** the at least one sensor is a first downstream sensor (28) on the dialysis fluid outlet line (8a) for measuring the concentration of the first substance in the spent dialysis fluid.

6. The blood treatment device according to claim 5, **characterized in that** it also comprises a dialysis fluid processing unit (11) connected to the control unit (34).

7. The blood treatment device according to claim 6, **characterized in that** the evaluation unit (33) and the control unit (34) are suitable for performing the determination of the blood purification performance L1 for the first substance by the following method:

storing the concentration C1di1 of the first substance in the fresh dialysis fluid in the evaluation unit (33),
measuring the concentration C1do1 of the first substance in the spent dialysis fluid with the first downstream sensor (28) and storing C1do1 in the evaluation unit (33),
altering the concentration C1 di of the first substance in the fresh dialysis fluid by the dialysis fluid processing unit (11) on command by the control unit (34),
storing the altered concentration C1di2 of the first substance in the fresh dialysis fluid in the evaluation unit (33),
measuring the altered concentration C1do2 of the first substance in the spent dialysis fluid with the first downstream sensor (28) and storing C1do2 in the evaluation unit (33), and
determination of the blood purification performance L1 by the evaluation unit (33) on the basis of the concentrations C1di1, C1do1 and altered concentrations C1di2, C1do2 of the first substance in the fresh and spent dialysis fluid.

8. The blood treatment device according to claim 7, **characterized in that** the dialysis fluid processing unit (11) performs the change in the concentration C1di in the form of steps or a bolus.

9. The blood treatment device according to claim 7, **characterized in that** it also comprises a first upstream sensor (27) connected to the evaluation unit (33) and situated on the dialysis fluid inlet line (7b) for measuring the concentrations C1di1 and C1di2 in the fresh dialysis fluid.

10. The blood treatment device according to any one of the preceding claims, **characterized in that** it also comprises a second downstream sensor (48), which is situated on the dialysis fluid outlet line (8a) and is connected to the evaluation unit (33) for measuring the concentration C2do of the second substance in the spent dialysis fluid.

11. The blood treatment device according to claim 10, **characterized in that** the evaluation unit (33) is suitable for determining the concentration C2bi of the second substance in the blood flowing to the second chamber (4) on the basis of the measured concentration C2do of the second substance in the spent dialysis fluid and the stored concentration C2di of the second substance in the fresh dialysis fluid as well as the blood purification performance L2 determined for the second substance.

12. The blood treatment device according to any one of the preceding claims, **characterized in that** the first substance is sodium.

**Revendications**

1. Dispositif de traitement de sang comportant un élément purificateur de sang (1) divisé en deux chambres par une membrane semi-perméable (2) et dont la première chambre (3) fait partie d'un circuit de liquide de dialyse et la seconde chambre (4) fait partie d'un circuit sanguin extracorporel,
comportant une conduite d'acheminement de liquide de dialyse pour l'acheminement de liquide de dialyse frais vers la première chambre (3) et/ou dans le circuit sanguin,
une conduite d'évacuation de liquide de dialyse pour l'évacuation du liquide de dialyse usagé hors de la première chambre (3),
une unité de commande (34) pour commander le dispositif de traitement de sang,
une unité d'exploitation (33),
au moins un capteur (27, 28) relié à l'unité d'exploitation (33) au niveau d'au moins un circuit sur le circuit sanguin et le circuit de liquide de dialyse pour détecter la concentration d'une première substance qui peut traverser la membrane semi-perméable (2),
l'unité d'exploitation (33) étant apte, à partir des valeurs de mesure de l'au moins un capteur (27, 28) à déterminer la performance de purification du sang L1 de l'élément purificateur de sang pour la première substance,
**caractérisé en ce que**
l'unité d'exploitation (33) est en outre apte à déterminer la performance de purification de sang L2 pour une seconde substance, différente de la performance de purification de sang L1 pour la première substance, de l'élément purificateur de sang à partir de la performance de purification2 de sang L1 pour la première substance et que la seconde substance est du potassium, du glucose ou du calcium.

2. Dispositif de traitement de sang selon la revendication 1, **caractérisé en ce que** la performance de purification de sang L est la dialysance Deff réelle.

3. Dispositif de traitement de sang selon la revendication 2, **caractérisé en ce que** l'unité d'exploitation (33) est apte à déterminer, à partir de la dialysance D1 eff pour la première substance un coefficient réel d'échange de masse k0A1eff, en partant du rapport f entre le coefficient théorique d'échange de masse k0A2th de la seconde substance et le coefficient théorique d'échange de masse k0A1th de la première substance, par multiplication avec k0A1eff, le coefficient réel d'échange de masse k0A2eff pour la seconde substance et à dériver de k0A2eff la dialysance réelle D2eff pour la seconde substance

4. Dispositif de traitement de sang selon la revendication 2, **caractérisé en ce que** l'unité d'exploitation (33) est apte à dériver du coefficient théorique d'échange de masse enregistré k0A1th pour la première substance et k0A2th D2eff pour la seconde substance des valeurs correspondant à celles-ci pour les dialysances théoriques D1th et D2th et à déterminer la dialysance réelle D2eff pour la seconde substance à partir de la dialysance mesurée D1 eff pour la première substance multipliée au ratio entre D2th et D1th.

5. Dispositif de traitement de sang selon une des revendications précédentes, **caractérisé en ce que** l'au moins un capteur est un premier capteur aval (28) placé au niveau de la conduite d'évacuation de liquide de dialyse (8a) pour la mesure de la concentration de première substance dans le liquide de dialyse usagé.

6. Dispositif de traitement de sang selon la revendication 5, **caractérisé en ce qu'**il comprend en outre une unité de préparation de liquide de dialyse (11) rattachée à l'unité de commande (34).

7. Dispositif de traitement de sang selon la revendication 6, **caractérisé en ce que** l'unité d'exploitation (33) et l'unité de commande (34) sont aptes à procéder à la détermination de la performance de purification de sang L1 pour la première substance par le procédé suivant :

enregistrement de la concentration C1di1 de première substance dans le liquide de dialyse frais dans l'unité d'exploitation (33),
mesure de la concentration C1do1 de première substance dans le liquide de dialyse usagé à l'aide du premier capteur aval (28) et enregistrement de C1do1 dans l'unité d'exploitation (33),
modification de la concentration C1di de première substance dans le liquide de dialyse frais par l'unité de préparation de liquide de dialyse (11) sur impulsion de l'unité de commande (34),
enregistrement de la concentration modifiée C1di2 de première substance dans le liquide de dialyse frais dans l'unité d'exploitation (33),
mesure de la concentration modifiée C1do2 de première substance dans le liquide de dialyse usagé (28) à l'aide du premier capteur aval et enregistrement de C1do2 dans l'unité d'exploitation (33) et
détermination de la performance de purification de sang L1 à partir des concentrations C1di1, C1do1 et des concentrations modifiées 1di2, C1do2 de première substance dans le liquide de dialyse frais et usagé (33) par l'unité d'exploitation (33).

8. Dispositif de traitement de sang selon la revendication 7, **caractérisé en ce que** l'unité de préparation de liquide de dialyse (11) effectue la modification de la concentration de C1di par étapes ou par bolus.

9. Dispositif de traitement de sang selon la revendication 7, **caractérisé en ce qu'**il comprend en outre un premier capteur amont (27) rattaché à l'unité d'exploitation (33) et situé au niveau de la conduite d'acheminement de liquide de dialyse (7b) pour la mesure des concentrations C1di1 et C1di2 dans le liquide de dialyse frais.

10. Dispositif de traitement de sang selon une des revendications précédentes, **caractérisé en ce qu'**il comprend en outre un second capteur aval (48) rattaché à l'unité d'exploitation (33) et situé au niveau de la conduite d'évacuation de liquide de dialyse (8a) pour la mesure de la concentration C2do de seconde substance dans le liquide de dialyse usagé.

11. Dispositif de traitement de sang selon la revendication 10, **caractérisé en ce que** l'unité d'exploitation (33) est apte à déterminer, à partir de la concentration mesurée C2do de seconde substance dans le liquide de dialyse usagé et de la concentration enregistrée C2di de seconde substance dans le liquide de dialyse frais et de la performance de purification de sang déterminée L2 de seconde substance, la concentration C2bi de seconde substance dans le sang affluant dans la seconde chambre (4) .

12. Dispositif de traitement de sang selon une des revendications précédentes, **caractérisé en ce que** la première substance est du sodium.

EP 1 615 680 B1

**IN DER BESCHREIBUNG AUFGEFÜHRTE DOKUMENTE**

**In der Beschreibung aufgeführte Patentdokumente**

- EP 0930080 A1
- DE 3938662 A1
- DE 19747360 A1
- WO 0002604 A1
- WO 9832476 A1
- EP 0658352 A1
- US 5567320 A
- EP 1062960 A2
- DE 10212247
- US 6126831 A

**In der Beschreibung aufgeführte Nicht-Patentliteratur**

- **J. Sargent ; F. Gotch et al.** Replacement of Renal Functions by Dialysis. Kluwer, 1996, 39ff
- **J. Sargent ; F. Gotch et al.** Replacement of Renal Functions by Dialysis. Kluwer, 1996, 41ff
- **H.D. Polaschegg ; N.W. Levin et al.** Replacement of Renal Functions by Dialysis. Kluwer, 1996, 371